# EUROPEAN PATENT SPECIFICATION

(11) **EP 3 207 970 B1**
(45) Date of publication and mention of the grant of the patent: **07.11.2018**
(21) Application number: 17151714.7
(22) Date of filing: 17.01.2017
(51) Int. Cl.: A63H 27/10, A61B 5/145

(54) **A FILLING CONTAINER**
ABFÜLLBEHÄLTER
RÉCIPIENT DE REMPLISSAGE

(30) Priority: 17.05.2016 CN 201620450162 U; 20.02.2016 CN 201620131314 U; 26.08.2016 CN 201620974741 U; 20.02.2016 CN 201620131221 U
(43) Date of publication of application: 23.08.2017
(73) Proprietor: Lin, Hui, Taizhou City Zhejiang (CN)
(72) Inventor: Lin, Hui, Taizhou City Zhejiang (CN)
(74) Representative: Keil & Schaafhausen Patent- und Rechtsanwälte PartGmbB

(56) References cited:
- WO-A1-2016/204828
- WO-A1-2016/204829
- CN-A- 104 436 683
- CN-A- 104 986 364
- CN-U- 204 193 466
- CN-U- 204 293 867
- CN-U- 205 198 964
- US-B1- 9 051 066

## Description

### Technical Field:

The current invention is related to the technical field of canned equipments, especially a new type of filling container.

### Technical Background:

It is well known that water fighting is a very popular entertainment for kids abroad and it requires a large amount of water. Since water fighting is very safe and entertaining, has a low budget, and a lot of people can participate at the same time, this activity has become more and more popular in China as well. However, the traditional way of making the water balls is very slow, and the balls are made individually, which is time consuming. The US patent, US 9051066, discloses a liquid filling container system and method. The system contains an opening at one side, and a shell body with several holes at the other side. The top parts of the hollow tubes correspond with each of the several holes respectively. The bottom parts of the hollow tubes are connected with a removable container.

The container is connected with the hollow tubes through elastic elements. Although with such a structure, more water balls can be made at the same time, it is a dispensable product and cannot be reused. CN 204293867 discloses a water ball filling device which comprises a sleeve pipe, balloons and water pipes. A water outlet in the lower end of the sleeve pipe is connected with the thin long water pipes. The tail ends of the water pipes are connected with the balloons. Threads are arranged in the sleeve pipe. A gasket is arranged in the sleeve pipe. The balloons and the water pipes are fixed through clamps. The tail ends of the balloons are provided with rubber bands.

### Content of the Invention:

The goal of the current invention is to provide a filling container, which has a simple structure, easy to use, highly efficient, highly air-tight, re-usable and can simultaneously produce multiple water balls or balloons or fill the balls with other liquids.

The goal of the invention is realized as follows:
A filling container, characterized in that it contains:
- a connector, wherein one side of the connector is provided with an entrance, whereas the other side of the connector is provided with a cavity for tubes, wherein the inside of the cavity for
- tubes is connected with said entrance;
- a bundle of hollow tubes containing a plurality of hollow tubes, wherein the top part of the bundle is inserted into the said cavity for tubes;
- containers, which are installed at the bottom part of hollow tubes through an elastic fixing element, wherein the inner cavity of each container is connected with the inner channels of the hollow tubes; and
- a constraining element, which is installed at the connector close to the cavity for tubes, wherein the constraining element shrinks the cavity for tubes and constrains the bundle of hollow tubes.

The bottom of connector is connected with a rubber sleeve, wherein the bottom side of the rubber sleeve is open and forms said cavity for tubes and the top part of the bundle of the hollow tubes inserts into the rubber sleeve; and the constraining element contains a tightening device which circles around the rubber sleeve, wherein the tightening device tightens the rubber sleeve and makes the rubber sleeve constrain the bundle of hollow tubes.

The tightening device is a ribbon, a rope, a rubber band or a hose hoop.

The hose hoop includes a hoop belt, a hoop shell and a locking worm, wherein the hoop shell is hollow and the inside of the hoop shell is a hole, the locking worm is located inside the hole of the hoop shell, the hoop shell is fixed on one side of the hoop belt, and the other side of the hoop belt extends through the hole of the hoop shell, and the perforations of the other side the hoop belt match with the screw thread of the locking worm, and the locking worm tightens the hoop belt so as the rubber sleeve, so that the rubber sleeve constrains the hollow tubes.

The outer wall of the rubber sleeve bends towards inside and forms a concave ring, wherein the tightening device is located inside concave ring.

The periphery of the bottom side of the rubber sleeve extends toward outside and forms protrusion.

One side of the connector extends toward outside and forms a slot, and the constraining element is a compressing block, wherein the compressing block can be removably installed inside the slot, and the slot and the compressing block form the cavity for tubes.

One side of the connector is provided with a sleeve, wherein the sleeve is provided with a plurality of tightening discs, and the tightening discs form said cavity for tubes; and the inside wall of the connector protrudes toward inside and forms a slope, and when the sleeve extends into the connector and tightens, the surface of slope resists against the tightening discs, so that the tightening discs lean toward inside and tightens the hollow tubes.

The hollow tubes comprise a plurality of hollow tubes whose cross sections are hexagon and form a bundle of hollow tubes which has a honeycomb shaped cross section, or the hollow tubes comprise a plurality hollow tubes which have circular cross sections.

Said elastic fixing element is a rubber or an elastic rubber ring or a clamp.

The current invention has the following advantageous technical effects in comparison with the prior art:
1. The filling container of the present invention can fill multiple balloons or containers simultaneously with water or liquid, so that multiple liquid balls or balloons can be made very fast, which solves the problem of producing liquid ball or balloon one by one in the prior art, which is very time consuming and inefficient. In addition, after the liquid ball and the balloon have been filled, new hollow tubes with container can be applied in order for the device to be re-used.
2. Through the rubber sleeve and the tightening device of the present invention, the installation of the hollow tubes is easier. When the tightening device is loosened, the hollow tube bundle can be inserted. The tightening device can be tightened afterwards, so that the rubber sleeve encloses the hollow tube bundle tightly. This method can be applied repeatedly. After the container has been filled, new hollow tubes with container can be used to replace the old ones. This method is convenient and fast, and also environment friendly.
3. Through the protruding flange of the present invention, the tightening device is not easy to escape from the outer surface of the rubber sleeve, and therefore the stability has been improved.
4. Through the concave ring of the present invention, the tightening device is not easy to escape from the outer surface of the rubber sleeve, and therefore the stability has been improved.
5. The hollow tubes comprise a plurality of hollow tubes whose cross sections are hexagon and form a bundle of hollow tubes which has a honeycomb shaped cross section. Or the hollow tubes comprise a plurality of hollow tubes whose cross sections are triangles. Or the hollow tubes comprise a plurality of hollow tubes whose cross sections are squares. In this way, there is no space between the hollow tubes, so that the airtightness is improved.
6. Through the compressing block of the present invention, the hollow tube bundle is easy to install. The compressing block can be removed when the tightening element is loosened, and the bundle of hollow tubes can be placed. The compressing block is then placed back in to press the hollow tubes and the compressing block is fixed by the fixing element. This method can be used repeatedly.

### Figures:

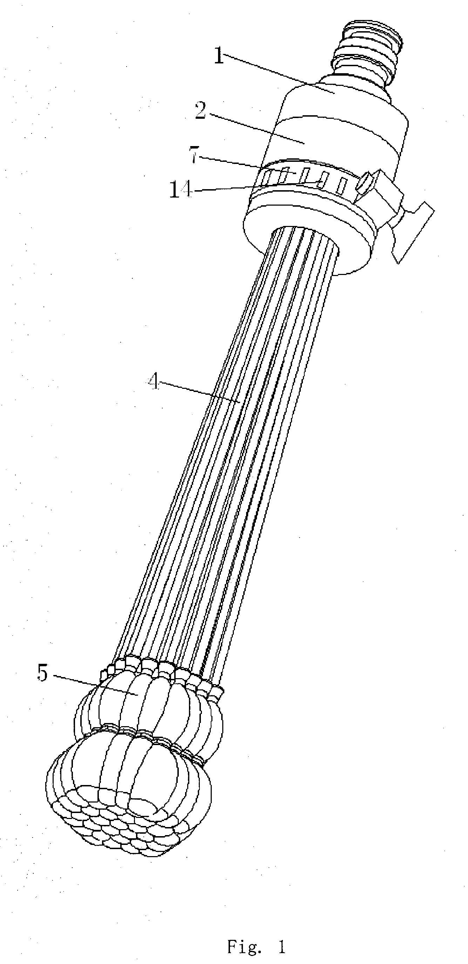

Fig. 1 shows the structure of the filling container of embodiment 1.

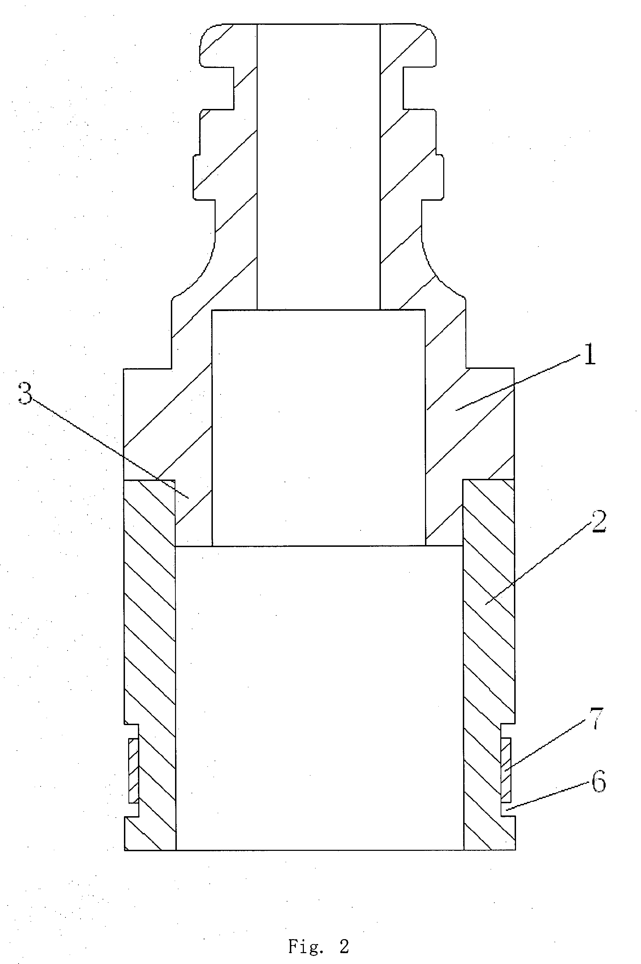

Fig. 2 shows the inside structure of the filling container of embodiment 1.

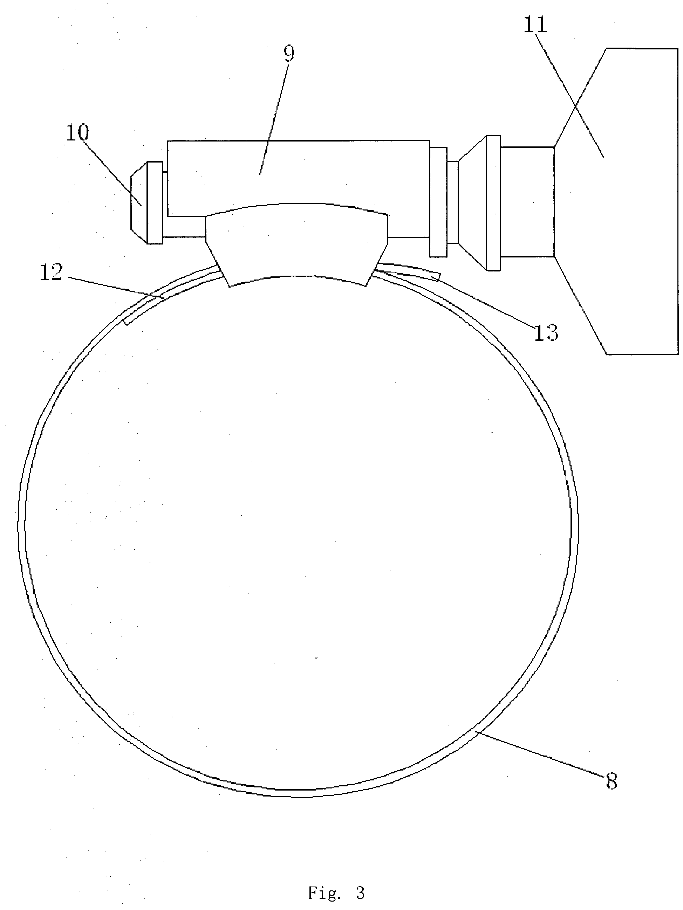

Fig. 3 shows the hose hoop of embodiment 1.

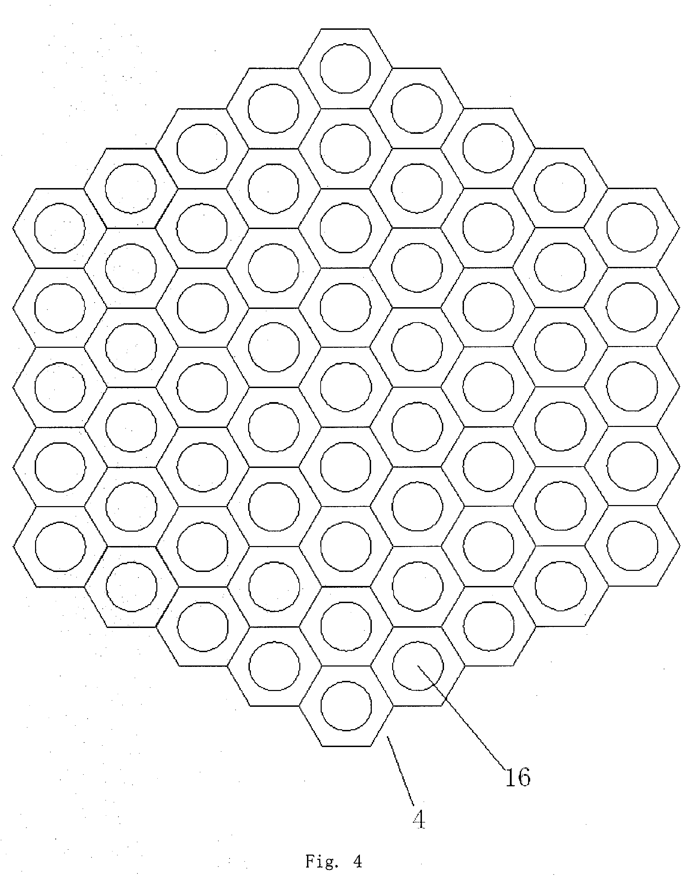

Fig. 4 shows the bundle of hollow tubes of embodiment 1.

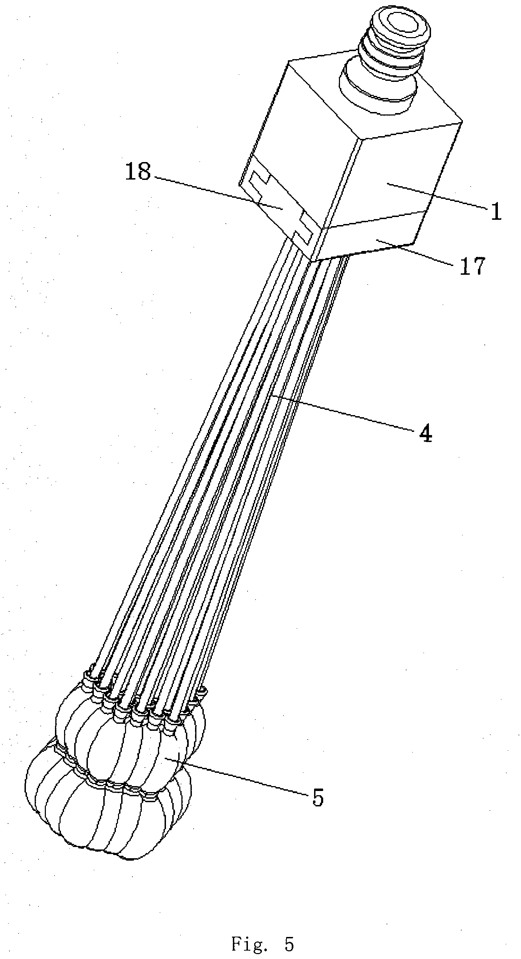

Fig. 5 shows structure 1 of the filling container of embodiment 2.

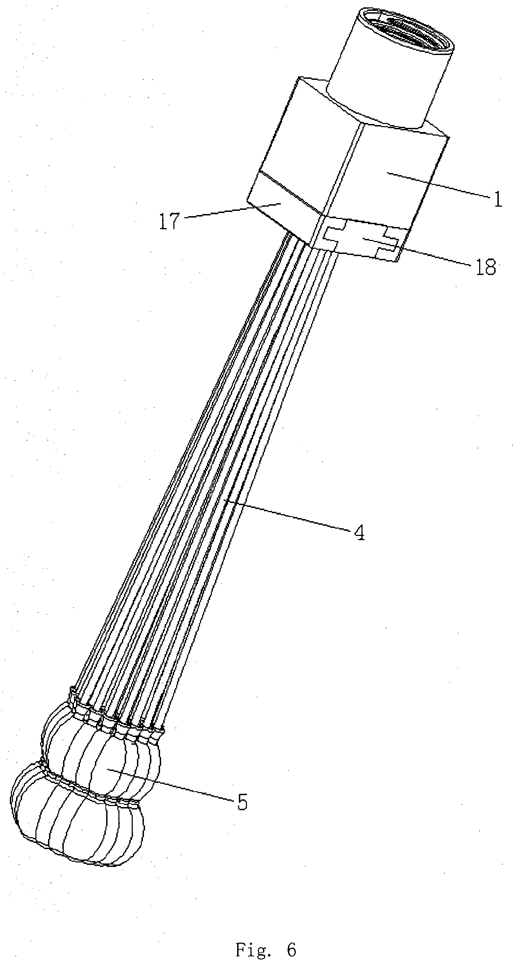

Fig. 6 shows structure 2 of the filling container of embodiment 2.

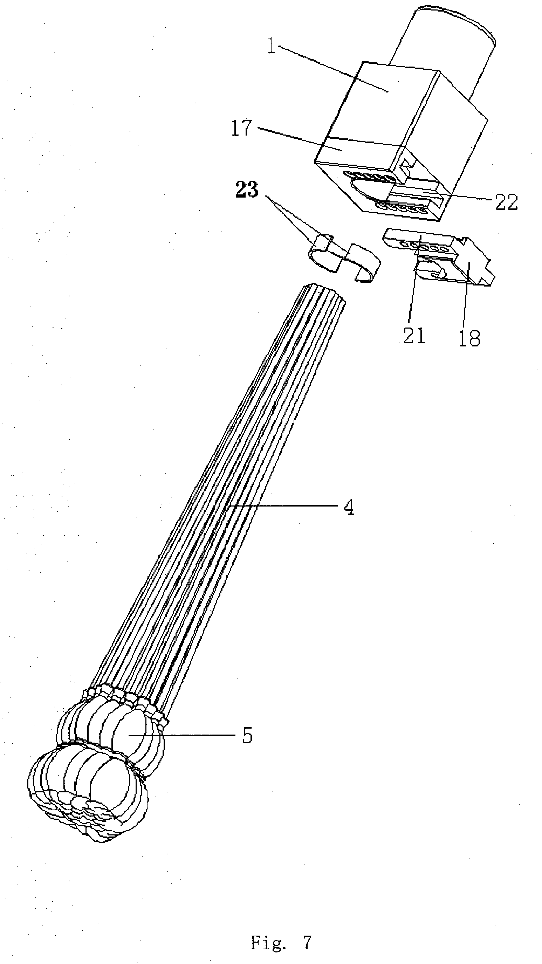

Fig. 7 shows the diagram of the components of the filling container of embodiment 2.

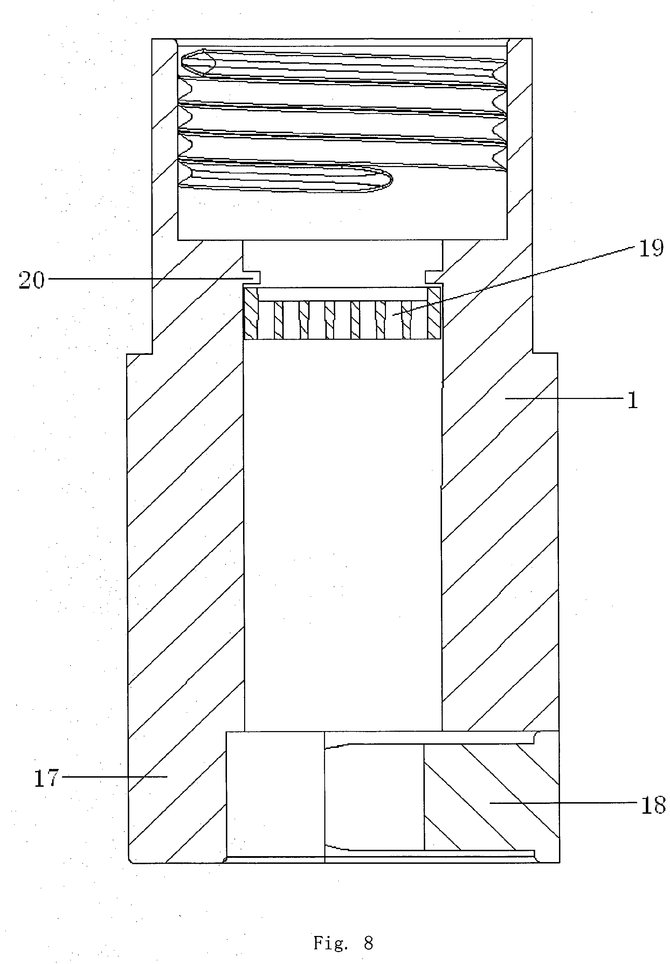

Fig. 8 shows the connector, slot, filtering block and the compressing block of embodiment 2.

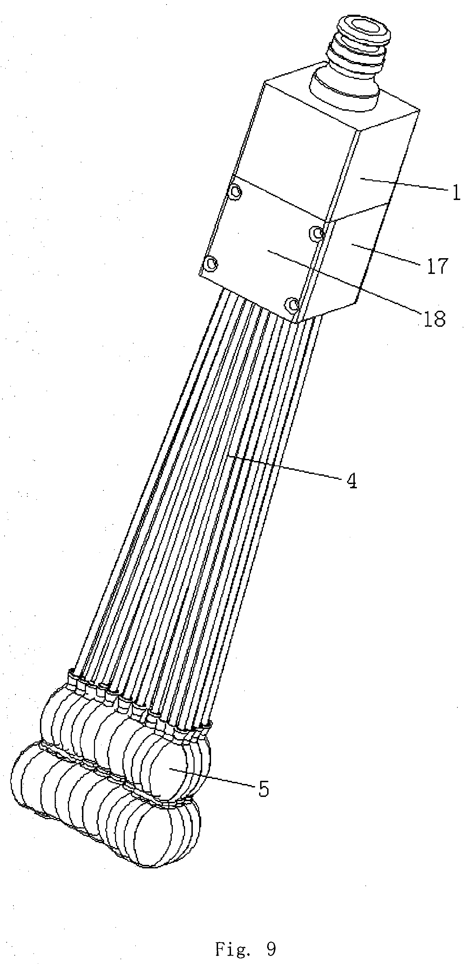

Fig. 9 shows structure 1 of the filling container of embodiment 3.

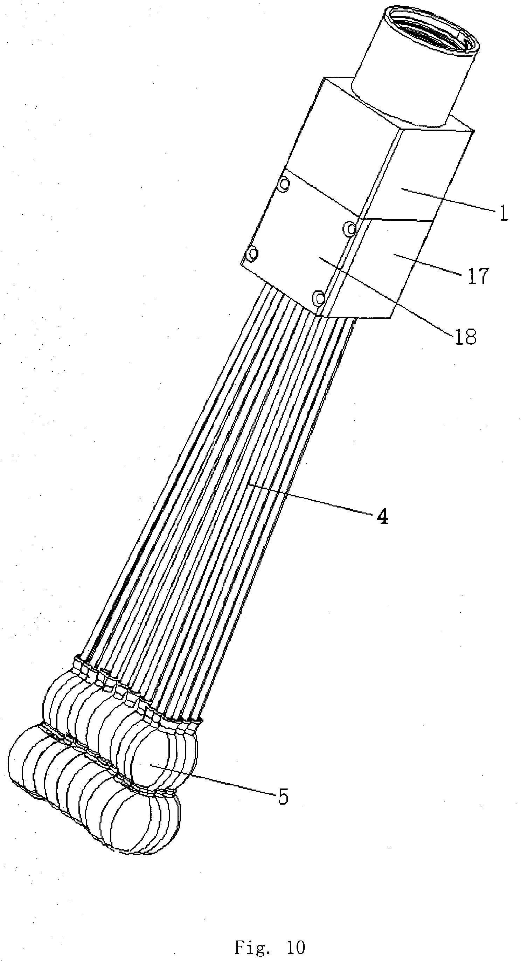

Fig. 10 shows structure 2 of the filling container of embodiment 3.

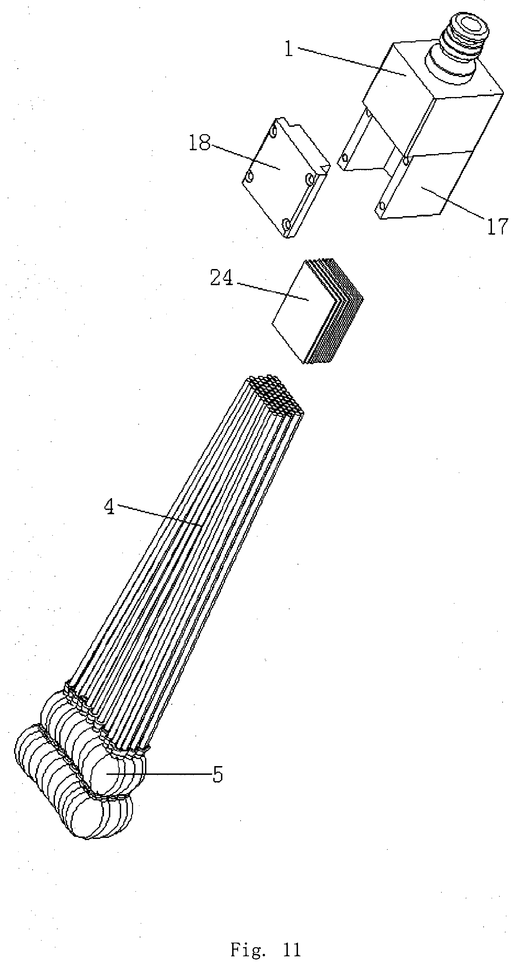

Fig. 11 shows the diagram of the components of the filling container of embodiment 3

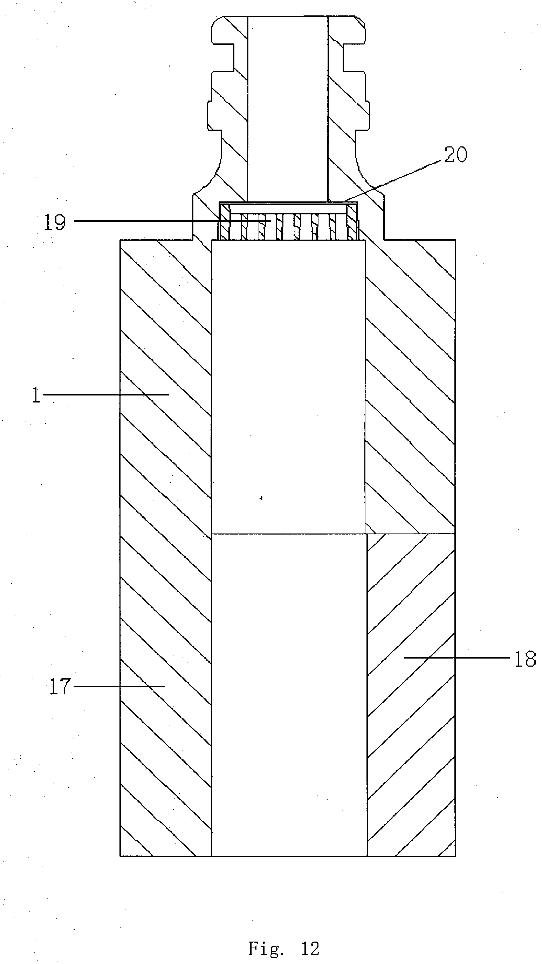

Fig. 12 shows the connector, slot, filtering block and the compressing block of embodiment 3.

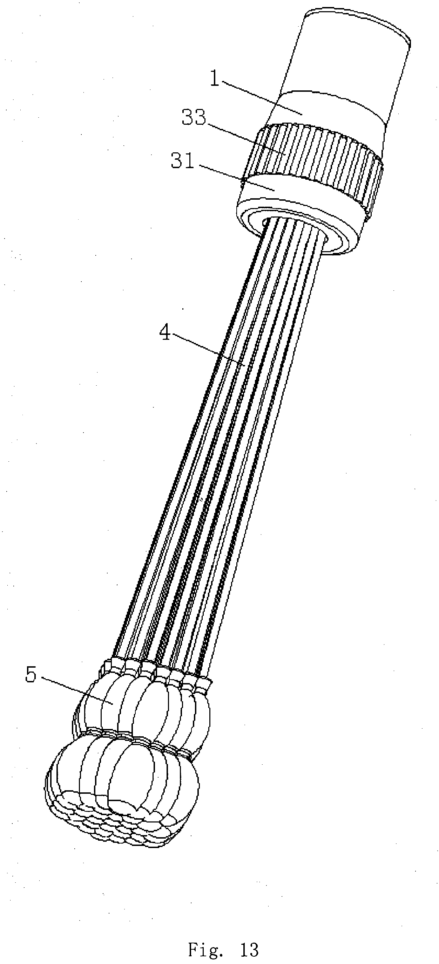

Fig. 13 shows structure 1 of the filling container of embodiment 4.

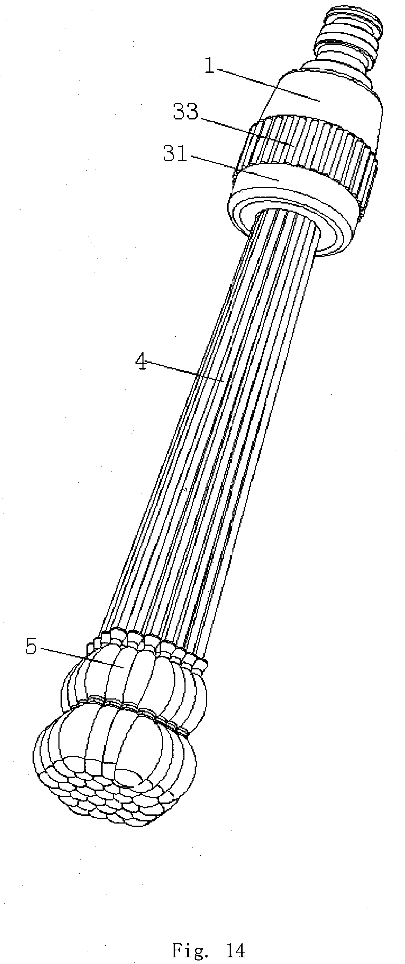

Fig. 14 shows structure 2 of the filling container of embodiment 4.

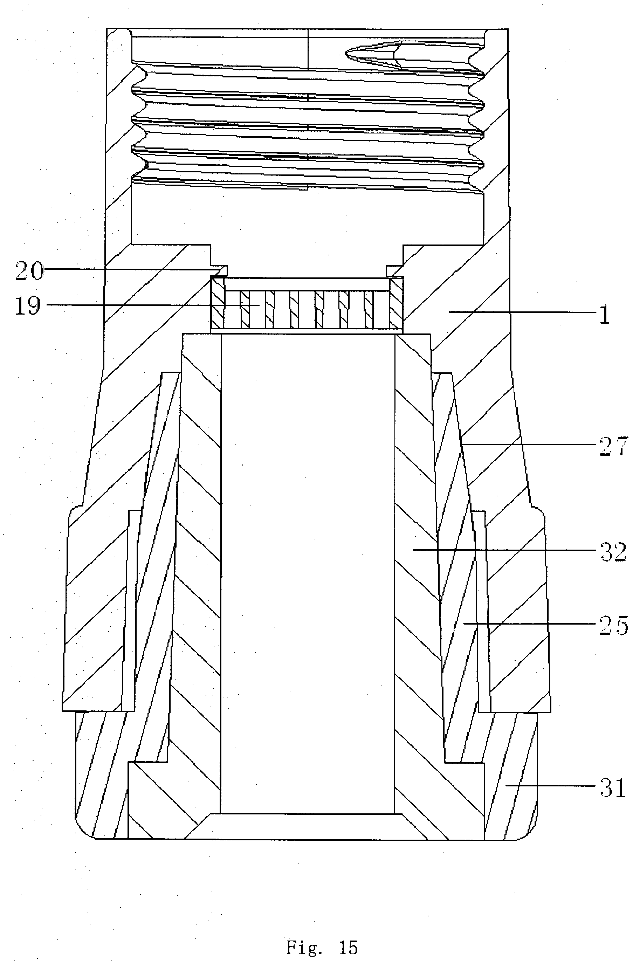

Fig. 15 shows diagram 1 of the connector, sleeve, and the sealing cushion of embodiment 4.

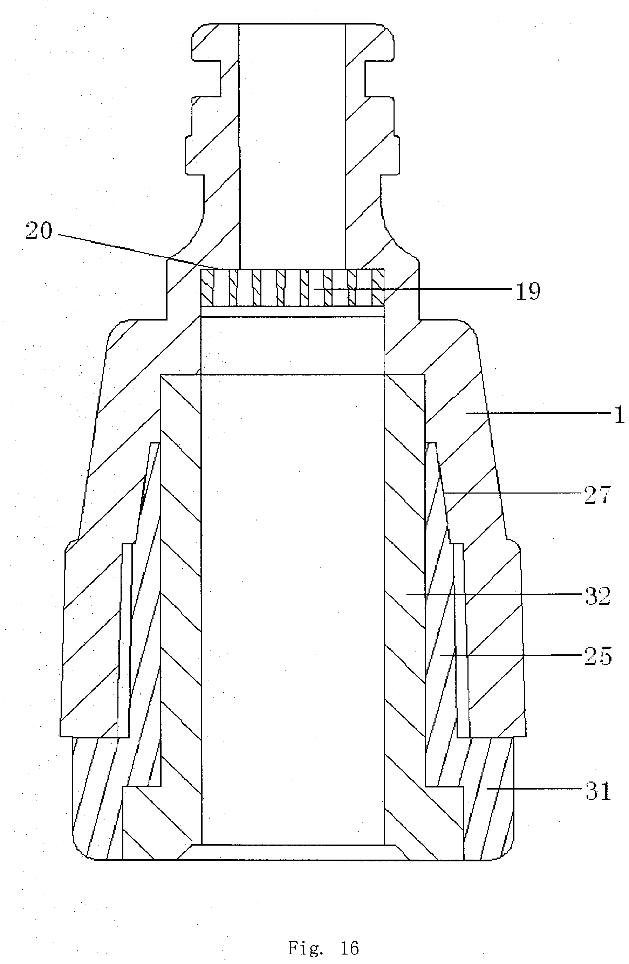

Fig. 16 shows diagram 2 of the connector, sleeve, and the sealing cushion of embodiment 4.

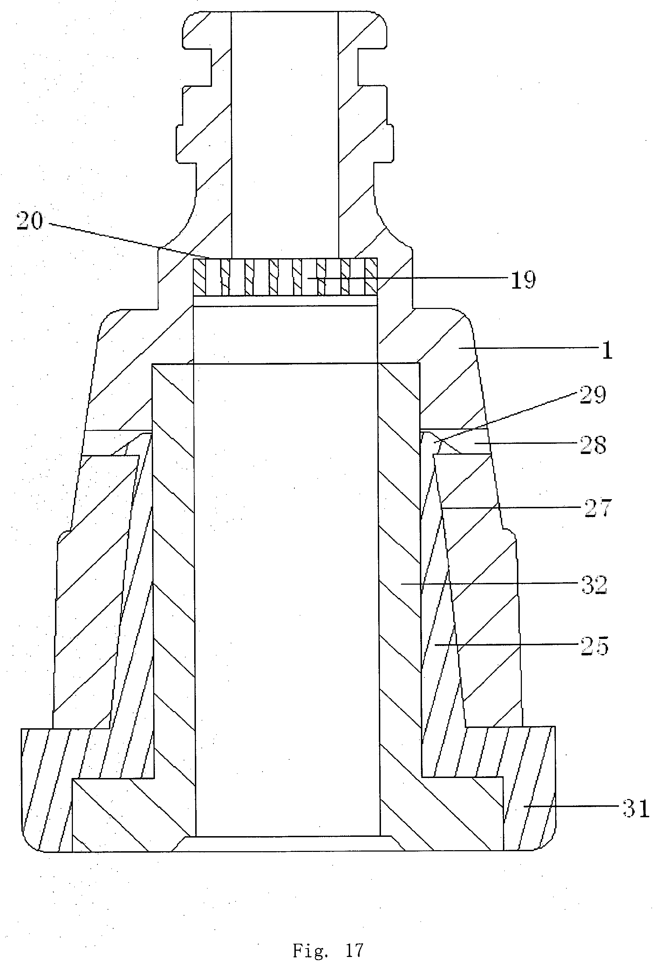

Fig. 17 shows the diagram of the connector, sleeve, and the sealing cushion of embodiment 5.

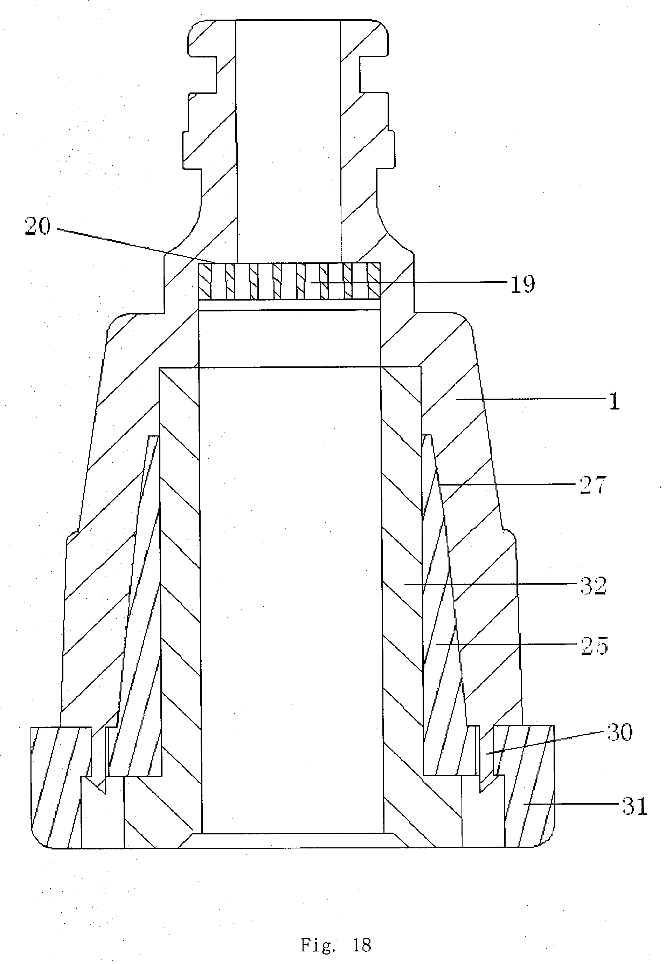

Fig. 18 shows the diagram of the connector, sleeve, and the sealing cushion of embodiment 6.

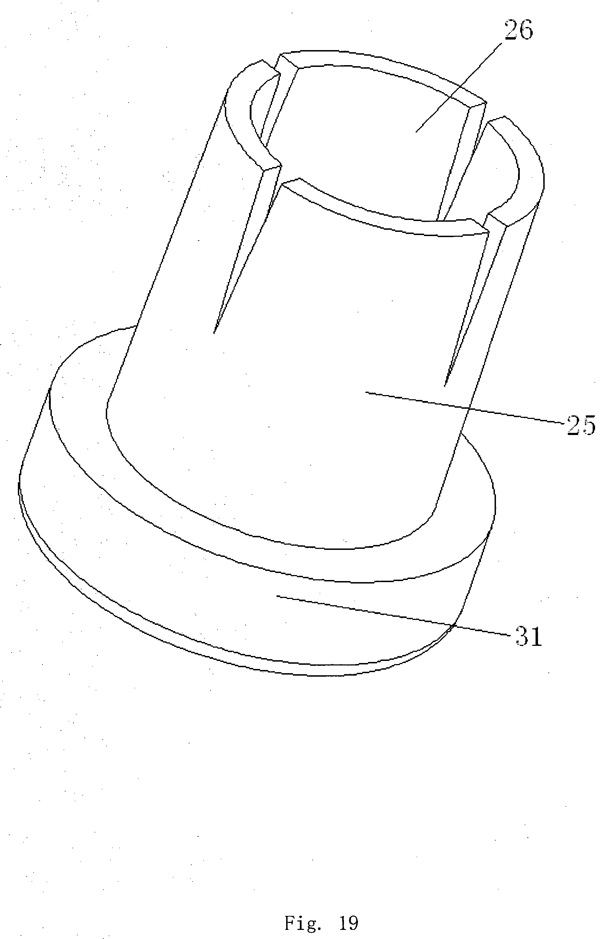

Fig. 19 shows the structure of the sleeve.

In the figures:
1- Connector; 2- rubber sleeve; 3- convex ring; 4-bundle of hollow tubes; 5-balloon; 6- concave ring; 7-hose hoop; 8- hoop belt; 9-hoop shell; 10- locking worm; 11-handle; 12-first end; 13-second end; 14-perforation; 16-hexagon hollow tube; 17-slot; 18-compressing block; 19-filtering block; 20-step; 21-fixing block; 22-chute; 23-sealing cushion II; 24-sealing cushion I, 25-sleeve; 26-tightening disc; 27-slope; 28-hole a; 29- block a; 30-block b; 31-protruding flange; 32-sealing cushion III; 33-anti slip strip.

### Embodiments:

The current invention is further illustrated by the following embodiments:
A filling container containing:
- a connector 1, wherein one side of the connector 1 is provided with an entrance, whereas the other side of the connector is provided with a cavity for tubes, wherein the inside of the cavity for tubes is connected with said entrance;
- a bundle of hollow tubes 4 containing a plurality of hollow tubes, wherein the top part of the bundle is inserted into the said cavity for tubes.

The bottom of connector 1 is connected with a rubber sleeve 2, wherein the bottom side of the rubber sleeve 2 is open and forms said cavity for tubes and the top part of the bundle of the hollow tubes 4 inserts into the rubber sleeve; and the constraining element contains a tightening device which circles around the rubber sleeve, wherein the tightening device tightens the rubber sleeve and makes the rubber sleeve constrain the bundle of hollow tubes. Preferably, the side of the connector 1, from which the water comes, extends downwards and forms a convex ring 3. The top part of the rubber sleeve 2 encloses the convex ring 3 and overlaps with the convex ring 3.

The containers are installed at the bottom part of hollow tubes through an elastic fixing element. The inner cavity of the container is connected with the inner channels of the hollow tubes.

Specifically, each container is a balloon 5 or a blood collecting bottle or a urine collecting bottle or other containers which are used to collect liquid or gas. When the containers are balloons 5 and when the water inside the balloon reaches certain weight, the balloon is basically full of water. Since the balloon full of water is heavier than the tightening force, the balloon falls off automatically. The elastic fixing element seals the balloon full of water automatically. Alternatively, when the balloon is full of water, the balloon can be taken off by hand. When the containers are blood collecting bottles or urine collecting bottles, the connector is connected with corresponding conducting tubes, which draw blood or urine from people or animals. Said connector can be an external connector, can internal connector, tubular connector or a spiral connector, as long as it can draw water and keep pressure.

The tightening device circles around the outer wall of the rubber sleeve 2, wherein the tightening device tightens the rubber sleeve and makes the rubber sleeve constrain the bundle of hollow tubes 4.

The hollow tubes 4 comprise a plurality of hollow tubes whose cross sections are hexagon and form a bundle of hollow tubes 4 which has a honeycomb shaped cross section.

Alternatively the hollow tubes 4 comprise a plurality hollow tubes which have circular cross sections.

The outer wall of the rubber sleeve 2 bends towards inside and forms a concave ring 6, wherein the tightening device is located inside concave ring 6, in order to prevent the tightening device from falling off. Alternatively, the bottom peripheral of the rubber sleeve extends outwards to form a retaining ring. The retaining ring retains the tightening device and prevents the tightening device from falling off.
The tightening device is a ribbon, a rope, a rubber band or a hose hoop 7.

The hose hoop 7 includes a hoop belt 8, a hoop shell 9 and a locking worm 10, wherein the hoop shell 9 is hollow and the inside of the hoop shell is a hole, the locking worm 10 is located inside the hole of the hoop shell 9, the hoop shell 9 is fixed on one side 12 of the hoop belt 8, and the other side 13 of the hoop belt 8 extends through the hole of the hoop shell 9, and the perforations 14 of the other side the hoop belt 8 match with the screw thread of the locking worm 10, and the locking worm 10 tightens the hoop belt so as the rubber sleeve, so that the rubber sleeve constrains the hollow tubes 4. Specifically, the tail of the locking worm extends backwards to form a handle 11. Through the handle, it is easier to execute forces, which facilitates operation.

Said connector is a pacifier connector.

Said elastic fixing element is a rubber or an elastic rubber ring or a clamp.

Said hollow tubes are flexible hollow tubes.

### Embodiment 2:

Embodiment 2 is partially identical with Embodiment 1 with the following differences:
One side of the connector extends toward outside and forms a slot 17, and the constraining element is a compressing block 18, wherein the compressing block 18 can be removably installed inside the slot 17, and the slot 17 and the compressing block 18 form the cavity for tubes.

The compressing surface of the compressing block has a shape of circular arc. The compressing surface and the side surface of the inner all of the slot form a circular opening. At this time, the hollow tubes have shapes corresponding to the shape of the opening. There is a sealing cushion II 23 between the several hollow tubes and the side wall of the opening. Part of the sealing cushion II 23 is fixed on the compressing surface of the compressing block, and other part is fixed on the inner wall of the slot 17. These two parts of the sealing cushion II 23 form a circular soft cushion.

The two sides of the compressing block are provided with a fixing block 21 respectively. The inner wall of the slot has a chute 22 which corresponds with the fixing block 21. The fixing block and the chute 22 are connected through a fixing element. Specifically, the fixing element is a screw or a bolt.

The inner side of the connector is connected with the entrance. The channel of the cavity for tubes is provided with a filtering block 19. The filtering block 19 is provided with several holes which extend from top to bottom. The filtering block is located above the hollow tubes. The shape of the filtering block 19 corresponds with the liquid channels. The filtering block 19 is connected with the side wall of the shell body with screws, or with cement, or clamps. The inside of the connector is connected with the entrance. The inner wall of the channel of the cavity for tubes has steps, wherein the top of the filtering block touches the step 20 and the bottom of the filtering block touches the top of the hollow tubes and stabilizes.

The compressing block 18 is connected with the shell body with screws or with cement or clamps. Preferably, the present invention uses screws.

### Embodiment 3:

Embodiment 3 is almost the same as embodiment 2 with the following differences:
The compressing surface of the compressing block 18 is a flat surface. The compressing surface and the inner side wall of the slot 17 form a square opening.

The hollow tubes 4 have several layers of hollow tubes. A sealing soft cushion I 24 is provided between the neighboring two layers of hollow tubes. Said layer of hollow tubes is consisted of several hollow tubes lying side by side. The cross section of the hollow tubes is circular or hexagon.

### Embodiment 4:

Embodiment 4 is almost the same as embodiment 2 with the following differences:
One side of the connector is provided with a sleeve 25, wherein the sleeve 25 is provided with a plurality of tightening discs 26, and the tightening discs 26 form said cavity for tubes. The inner wall of the connector 1 protrudes toward inside and forms a slope 27, and when the sleeve 25 extends into the connector 1 and tightens, the surface of slope 27 resists against the tightening discs 26, so that the tightening discs 26 lean toward inside and tightens the hollow tubes 4.

The connector 1 is screw connected with the sleeve 25. The top of the external wall of the tightening discs 26 is provided with external screw threads. The surface of the slope 27 and the bottom of the connector are provided with corresponding internal screw threads. When the shell body 17 and the sleeve 25 are screw tightened, the tightening discs 26 at the top of the sleeve 25 shrink inwards, so that the hollow tubes 4 are tightened.

A sealing cushion III 32 is provided between the hollow tubes and the side wall of the sleeve. The sealing cushion can be directly attached to the inner side of the sleeve. Said sealing soft cushion can be a ring or a sleeve.

The outer wall of the shell body has anti slip strip 33.

### Embodiment 5:

Embodiment 5 is almost the same as embodiment 4, with the following differences:
The sleeve 25 is clamping connected with the shell body 17.

The slope 27 of the inner wall of the shell body is provided with hole a 28. The outer surface of the tightening discs 26 is provided with a block a 29 corresponding with the hole a 28. When the shell body is enclosed into the sleeve, the tightening discs 26 shrink inwards and tighten the hollow tubes. The block a 29 touches the hole a 28 in order to prevent the sleeve 25 from falling off.

### Embodiment 6:

Embodiment 6 is almost the same as embodiment 5 with the following differences:
The sleeve 25 is clamping connected with the shell body 17.

The bottom part of the shell body extends downwards and forms several blocks b 30. The bottom part of the sleeve extends radially and forms the protruding flange 31. The protruding flange is provided with several blocks b 30 which correspond with the holes b. When the shell body is enclosed into the sleeve, the tightening discs 26 shrink inwards and tighten the hollow tubes. The block b 30 touches the hole b in order to prevent the sleeve from falling off.

The outer walls of the top part of each hollow tube of the bundle of hollow tubes are attached together and form an integral part. Alternatively, each hollow tube is independent to each other. The integral part means: the outer walls are glued together or are melted together to form one part.

The current invention has a simple structure, is easy to use and can make multiple water balls or balloons or fill the balls with other liquid at the same time. Its efficiency to make water ball or to fill the balls with other liquid is very high. It has high airtightness and can be reused.

## Claims

1. A filling container, containing:
- a connector (1), wherein one side of the connector (1) is provided with an entrance, whereas the other side of the connector (1) is provided with a cavity for tubes, wherein the inside of the cavity for tubes is connected with said entrance;
- a bundle of hollow tubes (4) containing a plurality of hollow tubes, wherein the top part of the bundle is inserted into the said cavity for tubes, and
- containers, which are installed at the bottom part of hollow tubes through an elastic fixing element, wherein the inner cavity of each container is connected with the inner channels of the hollow tubes;
**characterized in that** it further contains a constraining element, which is installed at the connector close to the cavity for tubes, wherein the constraining element shrinks the cavity for tubes and constrains the bundle of hollow tubes.

2. A filling container according to claim 1, **characterized in that** the bottom of connector (1) is connected with a rubber sleeve (2), wherein the bottom side of the rubber sleeve (2) is open and forms said cavity for tubes and the top part of the bundle of the hollow tubes (4) inserts into the rubber sleeve (2); and the constraining element contains a tightening device which circles around the rubber sleeve (2), wherein the tightening device tightens the rubber sleeve (2) and makes the rubber sleeve (2) constrain the bundle of hollow tubes (4).

3. A filling container according to claim 2, **characterized in that** the tightening device is a ribbon, a rope, a rubber band or a hose hoop (7).

4. A filling container according to claim 3, **characterized in that** the hose hoop (7) includes a hoop belt (8), a hoop shell (9) and a locking worm (10), wherein the hoop shell (9) is hollow and the inside of the hoop shell (9) is a hole, the locking worm (10) is located inside the hole of the hoop shell (9), the hoop shell (9) is fixed on one side (12) of the hoop belt (8), and the other side (13) of the hoop belt (8) extends through the hole of the hoop shell (9), and the perforations (14) of the other side (13) of the hoop belt (8) match with the screw thread of the locking worm (10), and the locking worm (10) tightens the hoop belt (8) so as the rubber sleeve (2), so that the rubber sleeve (2) constrains the hollow tubes.

5. A filling container according to claim 2, **characterized in that** the outer wall of the rubber sleeve (2) bends towards inside and forms a concave ring (6), wherein the tightening device is located inside concave ring (6).

6. A filling container according to claim 2, **characterized in that** the periphery of the bottom side of the rubber sleeve (2) extends toward outside and forms protrusion.

7. A filling container according to claim 1, **characterized in that** one side of the connector (1) extends toward outside and forms a slot, and the constraining element is a compressing block (18), wherein the compressing block (18) can be removably installed inside the slot, and the slot and the compressing block (18) form the cavity for tubes.

8. A filling container according to claim 1, **characterized in that** one side of the connector (1) is provided with a sleeve (25), wherein the sleeve (25) is provided with a plurality of tightening discs (26), and the tightening discs (26) form said cavity for tubes; and the inside wall of the connector (1) protrudes toward inside and forms a slope, and when the sleeve extends into the connector and tightens, the surface of slope (27) resists against the tightening discs (26), so that the tightening discs (26) lean toward inside and tightens the hollow tubes.

9. A filling container according to claim 2, **characterized in that** the hollow tubes comprise a plurality of hollow tubes whose cross sections are hexagon (16) and form a bundle of hollow tubes (4) which has a honeycomb shaped cross section, or the hollow tubes comprise a plurality hollow tubes which have circular cross sections.

10. A filling container according to any claim of claims 1 to 9, **characterized in that** the top of each hollow tube in the bundle form an integral part, or, each hollow tube is independent of each other; wherein the integral part means: the outer wall of the top of the hollow tubes are glued to each other via glue or are melted to each other.

## Patentansprüche

1. Ein Füllbehälter umfassend:
- einem Verbinder (1), wobei eine Seite des Verbinders (1) mit einem Eingang vorgesehen ist, während die andere Seite des Verbinders (1) mit einem Hohlraum für Röhren vorgesehen ist, wobei die Innenseite des Hohlraums für Röhren mit dem Eingang verbunden ist;
- ein Bündel hohler Röhren (4) umfassend mehrere hohle Röhren, wobei der obere Teil des Bündels in den Hohlraum für Röhren eingefügt ist, und
- Behälter, die durch ein elastisches Befestigungselement im unteren Teil der hohlen Röhren angebracht sind, wobei der innere Hohlraum jedes Behälters mit den inneren Kanälen der hohlen Röhren verbunden ist,
**dadurch gekennzeichnet, dass** der Füllbehälter ferner ein einschränkendes Element umfasst, das an dem Verbinder auf der Seite des Hohlraums für Röhren vorgesehen ist, wobei das einschränkende Element den Hohlraum für Röhren schrumpft und das Bündel von hohlen Röhren beschränkt.

2. Füllbehälter nach Anspruch 1, **dadurch gekennzeichnet, dass** der Boden des Verbinders (1) mit einer Gummihülse (2) verbunden ist, wobei der Boden der Gummihülse (2) offen ist und den Hohlraum für Röhren bildet, und der obere Teil des Bündels hohler Röhren (4) in der Gummihülse (2) eingefügt ist, und das einschränkende Element eine Spannvorrichtung umfasst, die die Gummihülse (2) umkreist, wobei die Spannvorrichtung die Gummihülse (2) anspannt, wodurch die Gummihülse (2) das Bündel hohlen Röhren (4) beschränkt.

3. Füllbehälter nach Anspruch 2, **dadurch gekennzeichnet, dass** die Spannvorrichtung ein Band, ein Seil, ein Gummiband oder eine Schlauchklemme (7) ist.

4. Füllbehälter nach Anspruch 3, **dadurch gekennzeichnet, dass** die Schlauchklemme (7) einen Gürtel (8), ein Gehäuse (9) und eine Verriegelungsschnecke (10) umfasst, wobei das Gehäuse(9) hohl ist und das Innere des Gehäuses (9) ein Durchgangsloch ist, die Verriegelungsschnecke (10) innerhalb des Durchgangslochs der Schale (9) vorgesehen ist, die Schale (9) auf einer Seite (12) des Gürtels (8) befestigt ist, und sich die andere Seite (13) des Gürtels (8) durch das Durchgangsloch der Schale (9) erstreckt, und die Perforierungen (14) der anderen Seite (13) des Gürtels (8) zum Gewinde der Verriegelungsschnecke (10) passen, und die Verriegelungsschnecke (10) den Gürtel (8) und somit die Gummihülse (2) anspannt, wodurch die Gummihülse (2) die hohlen Röhren einschränkt.

5. Füllbehälter nach Anspruch 2, **dadurch gekennzeichnet, dass** sich die Außenwand der Gummihülse (2) nach innen biegt und einen gewölbten Ring (6) bildet, wobei sich die Spannvorrichtung innerhalb des gewölbten Ring (6) befindet.

6. Füllbehälter nach Anspruch 2, **dadurch gekennzeichnet, dass** sich der Rand an der Unterseite der Gummihülse (2) nach außen erweitert und einen Vorsprung bildet.

7. Füllbehälter nach Anspruch 1, **dadurch gekennzeichnet, dass** sich eine Seite des Verbinders (1) nach außen ausweitet und einen Schlitz bildet, und das einschränkende Element ein komprimierender Block (18) ist, wobei der komprimierende Block (18) entfernbar im Schlitz installiert worden ist, und der Schlitz und der komprimierende Block (18) Hohlraum für Röhren bilden.

8. Füllbehälter nach Anspruch 1, **dadurch gekennzeichnet, dass** eine Seite des Verbinders (1) mit einer Hülse (25) versehen ist, wobei die Hülse (25) mit mehreren Spannscheiben (26) versehen ist, und die Spannscheiben (26) den Hohlraum für Röhren bilden, und wobei die Innenwand des Verbinders (1) nach innen vorsteht und eine Neigung bildet, und wenn sich die Hülse in den Verbinder erstreckt und anspannt, die Oberfläche (27) der Neigung einen Widerstand gegen die Spannscheiben (26) wirkt, sodass sich die Spannscheiben (26) nach innen neigen und die hohlen Röhren anspannen.

9. Füllbehälter nach Anspruch 2, **dadurch gekennzeichnet, dass** das Bündel hohler Röhren aus mehreren hohlen Röhren besteht, deren Querschnitte hexagonal (16) sind, wodurch ein Bündel hohlen Röhren gebildet wird, dessen Querschnitt honigwabenförmig ist, oder das Bündel hohlen Röhren aus mehreren hohlen Röhren besteht, deren Querschnitte kreisförmig sind.

10. Füllbehälter nach einem der Ansprüche 1 bis 9, **dadurch gekennzeichnet, dass** die Außenwände der Oberteile der hohlen Röhren des Bündels hohler Röhren einen integralen Bestandteil bilden oder dass jede hohle Röhre unabhängig von den anderen ist, wobei integraler Bestandteil bedeutet, dass die Außenwände der Oberteile der hohlen Röhren mit Klebstoff zusammengeklebt sind oder miteinander verschmolzen.

## Revendications

1. Récipient de remplissage, contenant :
- un connecteur (1), un côté du connecteur (1) étant pourvu d'une entrée tandis que l'autre côté du connecteur (1) est pourvu d'une cavité destinée à des tubes, l'intérieur de la cavité destinée aux tubes étant connecté à ladite entrée
- un faisceau de tubes creux (4) contenant une pluralité de tubes creux, la partie supérieure du faisceau étant insérée dans ladite cavité destinée aux tubes ; et
- des récipients qui sont installés à la partie inférieure des tubes creux par l'intermédiaire d'un élément de fixation élastique, la cavité interne de chaque récipient étant connectée aux canaux internes des tubes creux ;
**caractérisé en ce qu'**il comprend en outre un élément restrictif qui est installé au niveau du connecteur à proximité de la cavité destinée aux tubes, l'élément restrictif rétractant la cavité destinée aux tubes et contraignant le faisceau de tubes creux.

2. Récipient de remplissage selon la revendication 1, **caractérisé en ce que** le fond du connecteur (1) est connecté à un manchon en caoutchouc (2), la face inférieure du manchon en caoutchouc (2) étant ouverte et formant ladite cavité destinée aux tubes et la partie supérieure du faisceau de tubes creux (4) s'insérant dans le manchon en caoutchouc (2) ; et l'élément restrictif contenant un dispositif de serrage qui encercle le manchon en caoutchouc (2), le dispositif de serrage serrant le manchon en caoutchouc (2) et amenant le manchon en caoutchouc (2) à contraindre le faisceau de tubes creux (4).

3. Récipient de remplissage selon la revendication 2, **caractérisé en ce que** le dispositif de serrage est un ruban, une corde, une bande en caoutchouc ou un arceau de tuyau (7).

4. Récipient de remplissage selon la revendication 3, **caractérisé en ce que** l'arceau de tuyau (7) comprend une bande d'arceau (8), une coque d'arceau (9) et un serpentin de fermeture (10), la coque d'arceau (9) étant creuse et l'intérieur de la coque d'arceau (9) étant un trou, le serpentin de fermeture (10) étant situé dans le trou de la coque d'arceau (9), la coque d'arceau (9) étant fixée sur une face (12) de la bande d'arceau (8), et l'autre face (13) de la bande d'arceau (8) s'étendant à travers le trou de la coque d'arceau (9), et les perforations (14) de l'autre face (13) de la bande d'arceau (8) coïncidant avec le filetage du serpentin de fermeture (10), et le serpentin de fermeture (10) serrant la bande d'arceau (8) comme le manchon en caoutchouc (2), de manière à ce que le manchon en caoutchouc (2) contraigne les tubes creux.

5. Récipient de remplissage selon la revendication 2, **caractérisé en ce que** la paroi extérieure du manchon en caoutchouc (2) fléchit vers l'intérieur et forme un anneau concave (6), le dispositif de serrage étant situé dans l'anneau concave (6).

6. Récipient de remplissage selon la revendication 2, **caractérisé en ce que** la périphérie de la face inférieure du manchon en caoutchouc (2) s'étend vers l'extérieur et forme une saillie.

7. Récipient de remplissage selon la revendication 1, **caractérisé en ce qu'**une face du connecteur (1) s'étend vers l'extérieur et forme une fente, et que l'élément restrictif est un bloc de compression (18), le bloc de compression (18) pouvant être installé de manière amovible dans la fente, et la fente et le bloc de compression (18) formant la cavité destinée aux tubes.

8. Récipient de remplissage selon la revendication 1, **caractérisé en ce qu'**une face du connecteur (1) est pourvue d'un manchon (25), le manchon (25) étant pourvu d'une pluralité de disques de serrage (26), et les disques de serrage (26) formant ladite cavité destinée aux tubes ; et la paroi intérieure du connecteur (1) saillant vers l'intérieur et formant une pente et, lorsque le manchon s'étend dans le connecteur et procède au serrage, la surface de la pente (27) résiste aux disques de serrage (26), de sorte que les disques de serrage (26) fléchissent vers l'intérieur et serrent les tubes creux.

9. Récipient de remplissage selon la revendication 2, **caractérisé en ce que** les tubes creux comprennent une pluralité de tubes creux dont les sections transversales sont hexagonales (16) et forment un faisceau de tubes creux (4) qui a une section transversale en forme de nid d'abeilles, ou que les tubes creux comprennent une pluralité de tubes creux qui ont des sections transversales circulaires.

10. Récipient de remplissage selon l'une quelconque des revendications 1 à 9, **caractérisé en ce que** le sommet de chaque tube creux du faisceau forme une seule pièce ou que chaque tube creux est indépendant des autres, la notion de seule pièce signifiant : les parois extérieures du sommet des tubes creux sont collées les unes aux autres par de la colle ou sont fondues les unes avec les autres.
